(19) Europäisches Patentamt
European Patent Office
Office européen des brevets

(11) Veröffentlichungsnummer: **0 004 623**
**B1**

(12) # EUROPÄISCHE PATENTSCHRIFT

(45) Veröffentlichungstag der Patentschrift: **01.07.81**

(51) Int. Cl[3]: **C 07 C 85/04**

(21) Anmeldenummer: **79100904.6**

(22) Anmeldetag: **24.03.79**

(54) **Verfahren zur Herstellung von aromatischen Aminen.**

(30) Priorität: **06.04.78 DE 2814860**

(43) Veröffentlichungstag der Anmeldung: **17.10.79 Patentblatt 79/21**

(45) Bekanntmachung des Hinweises auf die Patenterteilung: **01.07.81 Patentblatt 81/26**

(84) Benannte Vertragsstaaten: **CH DE FR GB**

(56) Entgegenhaltungen:
**DE-A-2 719 365**
**CHEMICAL ABSTRACTS, Vol. 84, Nr. 5, 2. Februar 1976, Columbus, Ohio, USA, JONCZYK ANDRZEJ et al.: »Reactions of organic anions. LX Catalytic alkylation of some N-H acidic compounds in aqueous medium«, Zusammenfassung Nr. 30793n**
**W. P. WEBER et al.: »Phase transfer catalysis in organic synthesis«, 1977, Springer Verlag Berlin, Heidelberg, New York, Seiten 202—205**

(73) Patentinhaber: **BAYER AG, Zentralbereich Patente, Marken und Lizenzen, D-5090 Leverkusen 1, Bayerwerk (DE)**

(72) Erfinder: **Marhold, Albrecht, Dr., Carl-Duisberg-Strasse 329, D-5090 Leverkusen (DE)**
Erfinder: **Lantzsch, Reinhard, Dr., Heymann-Strasse 32, D-5090 Leverkusen (DE)**
Erfinder: **Klauke, Erich, Dr., Eichendorffweg 8, D-5068 Odenthal (DE)**
Erfinder: **Kysela, Ernst, Dr., Auf dem Kamm 17, D-5060 Bergisch-Gladbach 3 (DE)**

**EP 0 004 623 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von aromatischen Aminen

Die Erfindung betrifft ein Verfahren zur Herstellung von aromatischen Aminen durch Umsetzung aktivierter Halogenverbindungen mit wäßriger Ammoniaklösung bei erhöhter Temperatur.

Das Verfahren ist dadurch gekennzeichnet, daß man als aktivierte Halogenverbindungen solche der Formel (I)

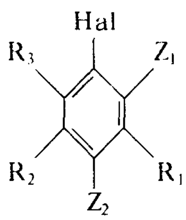

(I)

worin

Hal für Cl oder Br

$Z_1$ und $Z_2$ unabhängig voneinander für $NO_2$, H oder $CF_3$, jedoch nicht gleichzeitig für H und/oder $CF_3$,

$R_1$ und $R_2$ unabhängig voneinander für H, $C_1-C_4$-Alkyl, $C_1-C_4$-Alkoxy, Cl oder Br und

$R_3$ für $R_1$ bzw. $R_2$ oder $CF_3$

stehen, verwendet und man die Umsetzung in Gegenwart von Phasentransfer-Katalysatoren vornimmt.

Geeignete Phasentransferkatalysatoren zur Durchführung des erfindungsgemäßen Verfahrens sind Verbindungen der Formel (I)

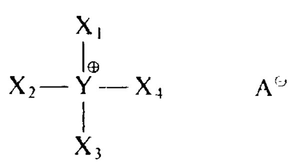

worin

$X_1-X_4$ unabhängig voneinander für einen gegebenenfalls substituierten Alkyl-, Cycloalkyl- oder Arylrest oder einen gegebenenfalls durch Divinylbenzol vernetzen Polystyrolrest,

Y für Stickstoff oder Phosphor und

$A^{(-)}$ für ein Anion stehen.

Besonders bewährt haben sich quartäre Ammoniumsalze der angegebenen Formel (Y=N), in der $X_1$, $X_2$ und $X_3$ unabhängig voneinander für $C_1-C_{18}$-Alkyl und $X_4$ für $C_1-C_4$-Alkyl, Benzyl oder Phenyl stehen.

Ganz besonders bevorzugt sind symmetrische quartäre Ammoniumsalze der angegebenen Formel, in der $X_1-X_4$ für $C_1-C_6$-Alkyl steht.

Geeignete Alkylreste $X_1-X_4$ sind z. B. $C_1-C_{18}$-Alkylreste wie der Methyl, Äthyl, Propyl, n-Butyl-, Hexyl-, Dodecyl und Octadecylrest und vor allem durch einen Hydroxy-, Cyan- oder Phenylrest substituierte $C_1-C_{18}$-Alkylreste, wie der 2-Hydroxyäthyl-, 2-Hydroxypropyl-, 2-Cyanäthyl- oder 2-Phenyläthylrest.

Als Cycloalkylreste $X_1-X_4$ kommen vor allem $C_5-C_6$-Cycloalkylreste, wie der Cyclopentyl und der Cyclohexylrest und die durch $C_1-C_4$-Alkylgruppen substituierten Cyclpentyl- und Cyclohexylreste, in Betracht.

Als geeignete Arylreste seien vor allem die durch $C_1-C_4$-Alkylreste substituierten Phenylreste genannt.

Als quartäre Ammoniumsalze, bei denen einer der Reste $X_1$ bis $X_4$ ein polymerer Rest ist, seien beispielsweise Ionenaustauscher auf der Basis von Styrol genannt, die im allgemeinen 2 Mol-% Divinylbenzol als Vernetzer, die quartäre Ammoniumgruppen tragen.

Als Anion $X^-$ kommen vor allem Halogenidionen (insbesondere $Cl^-$) sowie das Hydrogensulfation in Betracht.

Als Vertreter der erfindungsgemäß zu verwendenden Ammoniumsalze seien beispielsweise genannt:

Tetramethylammoniumchlorid,
Tetramethylammoniumjodid,
Tetraäthylammoniumchlorid,
Tetrabutylammoniumbromid,
Triäthylbenzylammoniumchlorid,
Trioctylmethylammoniumchlorid,
Benzyldodecyldimethylammoniumchlorid,
Tetrabutylammoniumhydrogensulfat.

Geeignete aktivierte Halogenverbindungen als Ausgangsmaterialien für das erfindungsgemäße Verfahren sind insbesondere solche der Benzolreihe, vorzugsweise solche der Formel

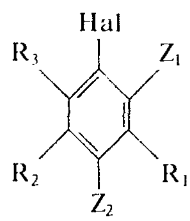

(II)

worin

Hal für Cl oder Br,

$Z_1$ und $Z_2$ unabhängig voneinander für $NO_2$, H oder $CF_3$, jedoch nicht gleichzeitig für H und/oder $CF_3$,

$R_1$ und $R_2$ unabhängig voneinander für H, $C_1-C_4$-Alkyl, $C_1-C_4$-Alkoxy, Cl oder Br und

$R_3$ für $R_1$ bzw. $R_2$ oder $CF_3$ stehen.

Diese Verbindungen sind bekannt bzw. in an sich bekannter Weise zugänglich.

Beispielhaft seien die folgenden Halogenverbindungen genannt:

2-Nitrochlorbenzol,
4-Nitrochlorbenzol,

2,4-Dinitrochlorbenzol,
4-Nitrofluorbenzol,
2,4-Di-trifluormethylchlorbenzol,
2-Nitro-4-trifluormethylchlorbenzol,
2-Trifluormethyl-4-nitrochlorbenzol,
2-Chlor-3-nitro-5-trifluormethyltoluol,
2-Chlor-5-nitro-3-trifluormethyltoluol,
3-Nitro-5-trifluormethyl-1,2-dichlorbenzol,
3-Nitro-2-chlor-5-trifluormethylbrombenzol,
2-Chlor-6-nitro-benzthiazol,
2,4-Dinitro-1-bromnaphthalin,
3-Phenyl-5-chlor-thiadiazol-(1,2,4).

Die für die Umsetzung erforderliche Menge an Phasentransferkatalysatoren kann in weiten Grenzen schwanken. Im allgemeinen haben sich Mengen von 0,1 bis 50 Gew.-%, vorzugsweise 1,0 bis 20 Gew.-%, bezogen auf das Gewicht des umzusetzenden Halogenids, bewährt.

In dem erfindungsgemäßen Verfahren wird ein Ammoniaküberschuß eingesetzt, üblicherweise die 5 bis 25fache Menge. Die Ammoniakkonzentration beträgt in etwa zwischen 15 und 60%, vorzugsweise 25 und 50%.

Die ideale Reaktionstemperatur kann für jedes eingesetzte Halogenid je nach Reaktivität, Reaktionszeit und Katalysatormenge durch wenige Versuche leicht ermittelt werden. Sie liegt im allgemeinen zwischen 80 und 200°C, vorzugsweise zwischen 110 und 160°C.

Die Durchführung der Reaktion erfolgt in üblicher Weise (vergleiche z. B. Houben-Weyl, »Methoden der Org. Chem.«, Band XI/1, Seite 63 f).

Es ist als ausgesprochen überraschend anzusehen, daß die Reaktion so glatt verläuft, da aus der gleichen Literaturstelle (vgl. Seite 31 in Kombination mit Seite 30) bekannt war, daß bei der Arylierung von Ammoniak — im Gegensatz zur Alkylierung — die erfindungsgemäß bevorzugt zu verwendenden Ammoniumsalze reaktionsverzögernd wirken.

Gegenüber den bekannten, ohne Transferkatalysatoren arbeitenden Aminolysemethoden weist das erfindungsgemäße Verfahren eine Reihe wichtiger Vorteile auf.

In erster Linie ist dabei die Herabsetzung der Reaktionstemperaturen zu nennen, wodurch häufig erstmals die Durchführung derartiger Reaktionen im großtechnischen Maßstab ermöglicht wird.

Auf jeden Fall wird das Sicherheitsrisiko erheblich vermindert, da der Abstand zwischen Reaktionstemperatur und Zersetzungstemperatur der Reaktionsprodukte oftmals wesentlich vergrößert wird.

Ein weiterer Vorteil des erfindungsgemäßen Verfahrens ist eine sehr starke Verminderung der Korrosion, die durch das Arbeiten bei niedrigerer Temperatur erreicht wird.

Die Verfahrensprodukte sind wertvolle Ausgangsmaterialien zur Herstellung von Farbstoffen, Pflanzenschutz- und Arzneimitteln.

So lassen sich die diazotierbaren Amine in bekannter Weise in anwendungstechnisch hochwertige Azofarbstoffe überführen.

o-Nitranilin kann darüber hinaus z. B. zu o-Phenylendiamin reduziert werden, das seinerseits ein Ausgangsstoff zur Synthese von bekannten Benzimidazolgruppenhaltigen Fungiziden oder Cumarinfarbstoffen ist.

## Beispiel 1

In einem 1,3-l-Rührautoklaven werden 79 g o-Nitrochlorbenzol, 630 g 25%iges wäßriges Ammoniak und 12,5 g Tetraäthylammoniumchlorid 10 h auf 150°C erhitzt. Nach dem Abkühlen wird entspannnt und mit verdünnter $H_2SO_4$ auf pH 8 gestellt. Dann wird dreimal mit insgesamt 400 ml $CH_2Cl_2$ extrahiert, die organische Phase abgetrennt und das Lösungsmittel abdestilliert. Das zurückbleibende o-Nitranilin wird getrocknet. Der Gehalt an o-Nitranilin beträgt 98,2%, die Ausbeute ist nahezu quantitativ.

## Beispiel 2
### Vergleichsbeispiel

Beispiel 1 wird ohne Zusatz von Tetraäthylammoniumchlorid wiederholt. Das entstehende Produkt besteht lt. gaschromatographischer Analyse zu 67% aus unumgesetzten o-Nitrochlorbenzol und nur zu 33% aus o-Nitranilin.

## Beispiel 3

Beispiel 1 wird mit 14 g Tetrabutylammoniumchlorid statt mit Tetraäthylammoniumchlorid wiederholt. Das zurückbleibende o-Nitranilin ist 97%ig, die Ausbeute beträgt 96% d. Th.

## Beispiel 4

In einem 0,3-l-Rührautoklav werden 68 g 2-Trifluormethyl-4-nitro-chlorbenzol, 40 g Wasser und 4 g Tetraäthylammoniumchlorid vorgelegt und anschließend 50 g flüssiger Ammoniak aufgedrückt. Man erhitzt auf 130°C und rührt für 5 h bei dieser Temperatur. Nach dem Abkühlen wird entspannt und das Material auf eine Filternutsche ausgetragen. Nach dem Absaugen verbleibt ein kristallines Produkt, das mit je 30 ml Wasser zweimal gewaschen wird. Nach Ablauf des Waschwassers wird mit 20 ml 10%iger Salzsäure gewaschen. Es verbleibt ein gelbgrünes Produkt, das nach dem Trocknen 62 g wiegt. Das entstandene 2-Trifluor-4-nitro-anilin ist ca. 98%ig (97—98% d. Th.) und schmilzt bei 88—89°C.

Beispiel 5

In einem Autoklav werden 30 g (0,1 Mol) 2-Chlor-5-nitro-1,3-bistrifluormethylbenzol, 20 ml Wasser und 1,5 g Tetraäthylammoniumchlorid vorgelegt und anschließend 25 ml flüssiger Ammoniak aufgedrückt. Unter Rühren wird für 6 Stunden auf 130°C unter einem Eigendruck von 15 bar erhitzt. Nach dem Abkühlen und Entspannen wird das kristalline Rohprodukt abfiltriert und mit Wasser gewaschen. Nach Umkristallisieren aus wäßrigem Isopropanol erhält man 17 g 4-Nitro-2,6-bistrifluormethylanilid mit einem Schmelzpunkt von 147—148°C.

Beispiel 6

In einem 100-ml-Autoklaven werden 26 g (0,1 Mol) 2,3-Dichlor-5-nitro-benzotrifluorid zusammen mit 20 ml Wasser und 1,5 g Tetraäthylammoniumchlorid vorgelegt und 25 ml $NH_3$ flüssig aufgedrückt. Während der 6 Stunden Reaktionszeit bei 125°C fällt der Druck von 12 auf 10 bar. Nach Abkühlen und Entspannen wird der Ansatz zweimal mit je 50 ml Chloroform extrahiert. Die GC-Analyse zeigt an, daß sich das Ausgangsmaterial zu 91% zum gewünschten 2-Chlor-4-nitro-4-trifluormethylanilin umgesetzt hat. Durch Abdampfen des Lösungsmittels und Kristallisation aus wäßrigem Isopropanol erhält man 15 g Amin (F: 109—11°C).

Beispiel 7

Zu 4 g Tetraäthylammoniumchlorid in 40 ml Wasser gibt man 78 g (0,3 Mol) 2,4-Dichlor-5-nitro-benzotrifluorid in einen Autoklav und drückt 50 g flüssiges Ammoniak auf. Unter einem Eigendruck von anfangs 23 bar wird der Ansatz für 5 Stunden bei 130°C gerührt, abgekühlt und entspannt. Das kristalline Reaktionsprodukt wird abfiltriert und mit Wasser gewaschen. Das Reaktionsprodukt wird durch Destillation gereinigt. Man erhält bei Kp 140—45°C 44 g 2-Nitro-4-trifluormethyl-5-chloranilin mit einem Schmelzpunkt von 108—110°C.

Beispiel 8

Man legt in einem Autoklav 4 g Tetramethylammoniumchlorid, 40 ml Wasser und 68 g 2-Trifluormethyl-4-nitrochlorbenzol vor, drückt 50 g flüssiges Ammoniak auf und erhitzt für 5 Stunden auf 130°C. Die Reaktionsmischung wird in Chloroform aufgenommen, von der wäßrigen Phase getrennt und nach dem Trocknen über Natriumsulfat einer GC-Analyse unterworfen. Danach ist 2-Trifluormethyl-4-nitro-anilin zu 95% entstanden.

Beispiel 9

68 g 2-Trifluormethyl-4-nitro-chlorbenzol werden zusammen mit 46 g 20%iges wäßriges Ammoniak im Autoklav vorgelegt und dann 19 g flüssiges Ammoniak aufgedrückt. Man erhitzt für 5 Stunden auf 130°C. Der Anfangsdruck von 15 bar fällt auf 10 bar ab. Nach vorgenannter Aufarbeitung zeigt die GC-Analyse einen Umsatz von 34,8% an.

Beispiel 10

Man wiederholt den Versuch aus Beispiel 9 und setzt zur Reaktionsmischung noch 4 g Tetraäthylammoniumchlorid zu. Nach gleicher Aufarbeitung zeigt die GC-Analyse einen Umsatz von 91,2% an.

**Patentansprüche**

1. Verfahren zur Herstellung von aromatischen Aminen durch Umsetzung aktivierter Halogenverbindungen mit wäßriger Ammoniaklösung bei erhöhter Temperatur, dadurch gekennzeichnet, daß man als aktivierte Halogenverbindungen solche der Formel

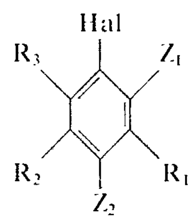

worin

| | |
|---|---|
| Hal | für Cl oder Br |
| $Z_1$ und $Z_2$ | unabhängig voneinander für $NO_2$, H oder $CF_3$, jedoch nicht gleichzeitig für H und/oder $CF_3$, |
| $R_1$ und $R_2$ | unabhängig voneinander für H, $C_1-C_4$-Alkyl, $C_1-C_4$-Alkoxy, Cl oder Br und |
| $R_3$ | für $R_1$ bzw. $R_2$ oder $CF_3$ |

stehen, verwendet und man die Umsetzung in Gegenwart von Phasentransfer-Katalysatoren vornimmt.

2. Verfahren nach Anspruch 1, dadurch gekennzeichnet, daß man als Transfer-Katalysatoren quartäre Ammonium- oder Phosphoniumsalze verwendet.

3. Verfahren nach Anspruch 1, dadurch gekennzeichnet, daß man als Phasentransfer-Katalysatoren Verbindungen der Formel

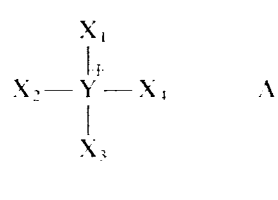

worin

$X_1-X_4$ unabhängig voneinander für einen gegebenenfalls substituierten Alkyl-, Cycloalkyl- oder Arylrest oder einen gegebenenfalls durch Divinylbenzol vernetzten Polystyrolrest,

Y für Stickstoff oder Phosphor und

$A^{(-)}$ für ein Anion

stehen, verwendet.

4. Verfahren nach Anspruch 1, dadurch gekennzeichnet, daß man als Halogenverbindung 2-Trifluormethyl-4-nitrochlorbenzol einsetzt.

5. Verfahren nach Anspruch 1, dadurch gekennzeichnet, daß man als Halogenverbindung 2-Nitro-4-trifluormethylchlorbenzol einsetzt.

**Claims**

1. Process for the preparation of aromatic amines by reacting activated halogen compounds with aqueous ammonia solution at elevated temperature, characterised in that the activated halogen compounds used are those of the formula

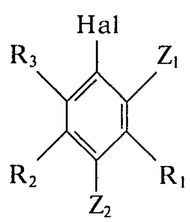

wherein

Hal represents Cl or Br

$Z_1$ and $Z_2$ independently of one another represent $NO_2$, H or $CF_3$, but do not simultaneously represent H and/or $CF_3$,

$R_1$ and $R_2$ independently of one another represent H, $C_1-C_4$-alkyl, $C_1-C_4$-alkoxy, Cl or Br and

$R_3$ represents $R_1$ or $R_2$ or $CF_3$

and the reaction is carried out in the presence of phase transfer catalysts.

2. Process according to claim 1, characterised in that quaternary ammonium salts or phosphonium salts are used as the transfer catalysts.

3. Process according to claim 1, characterised in that compounds of the formula

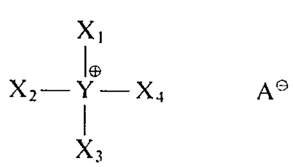

wherein

$X_1-X_4$ independently of one another represent an optionally substituted alkyl, cycloalkyl or aryl radical or a polystyrene radical optionally crosslinked by divinylbenzene,

Y represents nitrogen or phosphorus and

$A^{(-)}$ represents an anion,

are used as the phase transfer catalysts.

4. Process according to claim 1, characterised in that 2-trifluoromethyl-4-nitrochlorobenzene is employed as the halogen compound.

5. Process according to claim 1, characterised in that 2-nitro-4-trifluoromethylchlorobenzene is employed as the halogen compound.

**Revendications**

1. Procédé de production d'amines aromatiques par réaction de composés halogénés activés avec une solution aqueuse d'ammoniac à température élevée, caractérisé en ce qu'on utilise comme composés halogénés activés des composés de formule:

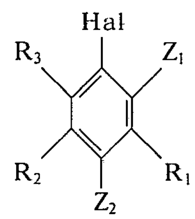

dans laquelle:

Hal représente Cl ou Br

$Z_1$ et $Z_2$ représentent, indépendamment l'un de l'autre, $NO_2$, H ou $CF_3$, mais ne représentent pas en même temps H et/ou $CF_3$

$R_1$ et $R_2$ représentent, indépendamment l'un de l'autre, H, un groupe alkyle en $C_1$ à $C_4$, un groupe alkoxy en $C_1$ à $C_4$, Cl ou Br et

$R_3$ représente $R_1$ ou $R_2$ ou un groupe $CF_3$

et on conduit la réaction en présence de catalyseurs de transfert de phase.

2. Procédé suivant la revendication 1, caractérisé en ce qu'on utilise comme catalyseurs de transfert des sels d'ammonium ou de phosphonium quaternaire.

3. Procédé suivant la revendication 1, caractérisé en ce qu'on utilise comme catalyseurs de transfert de phase des composés de formule:

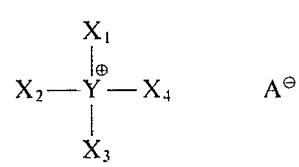

dans laquelle:

$X_1-X_4$ représentent, indépendamment l'un de

l'autre, un reste alkyle, cycloalkyle ou aryle éventuellement substitué ou un reste polystyrène éventuellement réticulé par du divinylbenzène

Y est de l'azote ou du phosphore et

$A^{(-)}$ est un anion.

4. Procédé suivant la revendication 1, caractérisé en ce qu'on utilise le 2-trifluorométhyl-4-nitrochlorobenzène comme composé halogéné.

5. Procédé suivant la revendication 1, caractérisé en ce qu'on utilise le 2-nitro-4-trifluorométhylchlorobenzène comme composé halogéné.